(19) Europäisches Patentamt
European Patent Office
Office européen des brevets

(11) Numéro de publication: 0 000 460
B1

(12) **FASCICULE DE BREVET EUROPEEN**

(45) Date de publication du fascicule de brevet: 04.11.81

(21) Numéro de dépôt: 78400048.1

(22) Date de dépôt: 03.07.78

(51) Int. Cl.³: B 01 J 23/50, C 07 D 301/10

# (54) Catalyseurs à base d'argent et leur utilisation pour la production d'oxyde d'oléfines.

(30) Priorité: 08.07.77 FR 7721118

(43) Date de publication de la demande: 24.01.79 Bulletin 79/2

(45) Mention de la délivrance du brevet: 04.11.81 Bulletin 81/44

(84) Etats Contractants Désignés: BE DE FR GB NL

(56) Documents cités: US - A - 3 899 445

(73) Titulaire: P C U K PRODUITS CHIMIQUES UGINE KUHLMANN
Service Propriété Industrielle Tour Manhattan
F-92087 PARIS LA DEFENSE 2 Cédex 21 (FR)

(72) Inventeur: Cognion, Jean Marie
85, Route de Charly
F-69230 St Genis Laval (FR)
Inventeur: Kervennal, Jacques
134, rue Docteur Locard
F-69005 Lyon (FR)

(74) Mandataire: Monceaux, Pierre et al,
PRODUITS CHIMIQUES UGINE KUHLMANN
Service Propriété Industrielle
Tour Manhattan Cédex 21 F-92087 Paris La Defense (FR)

EP 0 000 460 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Catalyseurs à base d'argent et leur utilisation pour la production d'oxyde d'oléfines

La présente invention concerne des catalyseurs à base d'argent sur support graphite pour l'époxydation en phase vapeur des oléfines et plus particulièrement pour la production, par cette technique, de l'oxyde d'éthylène à partir d'éthylène et d'oxygène moléculaire.

De façon générale la production d'oxyde d'éthylène est effectuée en phase vapeur, dans des réacteurs tubulaires à lit fixe, par réaction de l'oxygène et de l'éthylène sur des phases catalytiques à base d'argent déposées sur des supports réfractaires et inertes formés principalement d'alumine, de silice-alumine, de magnésie, de pierre ponce, de zircone, d'argile, de céramique, d'amiante, de zéolithe naturelle ou artificielle ou de carbure de silicium. L'art antérieur préfère généralement les supports à base d'alumine $\alpha$ possédant une surface spécifique inférieure à quelques m2/g. Cette surface spécifique est déterminée par la méthode d'adsorption de l'azote, méthode dite B.E.T. décrite par BRUNAUER, EMMET et TELLER dans "The journal of the american chemical Society" vol. 60, page 309, 1938. La deuxième caractéristique importante de ces supports est la porosite: elle est en général élevé: 30 à 60% en volume, et constitués de pores de rayons importants. C'est ainsi que l'on trouve cités dans les brevets français n° 2.006.849 et 2.253.747 des rayons de pores de 1 à 15 microns, dans le brevet français n° 2.117.189 des rayons de pores de 2 à 40 microns, dans le brevet français n° 2.243.193 des rayons de pores de 10 à 300 microns at enfin dans le brevet français n° 2.029.751 des rayons de pores de 200 à 1500 microns.

La demanderesse a découvert que l'époxydation catalytique des oléfines et en particulier celle de l'éthylène, pouvait être effectuée avec de bonnes sélectivités et avec des productivités supérieures à celles des catalyseurs décrits dans l'art antérieur par l'emploi comme supports de certains graphites, tels ceux récemment mis au point par la société. Le Carbone Lorraine et faisant l'objet du brevet français 2.315.482 du 24 Juin 1975 et de la demande de brevet français 77 14581 du 12 Mai 1977.

Les nouveaux supports graphités ou matériaux graphités sont caractérisés par:

. une parfaite résistance à l'oxydation

. une granulométrie suffisamment élevés, 50 microns à 10 mm permettant leur emploi dans les réacteurs industriels.

. des propriétés mécaniques satisfaisantes, évitant la formation de poussières au cours de la préparation du catalyseur, de ses manipulations ou de son fonctionnement.

. une surface spécifique faible, inférieure à 10m2/g de préférence comprise entre 0,1 et 2 m2/g.

. un volume de porosité de 0,1 à 0,4 cm3/g, réparti différemment suivant la granulométrie dans les domaines de rayons de pores inférieurs ou supérieurs à 6,6 microns. Pour les faibles granulométries, inférieures à 0,7 mm, la macroporosité, constituée de pores de rayons supérieurs à 6,6 microns peut être faible et une partie importante de la porosité, pouvant atteindre 90% de la porosité total peut être concentrée dans le domaine de rayons de pores de 50 A° à 6,6 microns. Pour les granulométries plus élevées il doit exister, à côté du domaine de microporosité précédemment décrit, une macroporosité formée de rayons de pores pouvant atteindre 200 microns. Cette macroporosité peut représenter 50 à 90% de la porosité totale.

Ces graphites artificiels poreux peuvent être obtennus sous des formes très différentes comme des sphères, des pastilles, des anneaux, des cassons ou des formes extrudées.

L'amélioration apportée par ce nouveau type de support est double, il permet d'obtenir avec des sélectivités meilleures des productivités en oxyde d'éthylène plus élevées. Cette amélioration peut être attribuée à la texture et à la structure du graphite mais aussi à la bonne conductibilité thermique de ces solides qui permet une élimination rapide des calories formées dans la zone réactionnelle. Cette élimination rapide des calories limite la réaction de dégradation thermique de l'oxyde d'éthylène en dioxyde de carbone et permet par conséquent des productivités élevées. Dans l'art antérieur, on trouve quelques tentatives d'emploi de supports bons conducteurs thermiques, mais il s'agit de solides qu'il est difficile d'obtenir avec une texture déterminée. On peut noter les métaux (brevet anglais n° 1.133.484), les ferrosiliciums (brevet allemand n° 1.093.344) la magnétite (brevet américain n° 2.593.156) le carbure de silicium (brevet anglais n° 1.133.484).

On trouve également mentionné l'emploi du graphite comme support de l'argent dans la synthèse de l'oxyde d'éthylène dans les brevets français n° 1.079.601 et anglais n° 775.218 ainsi que dans le brevet allemand n° 1.066.569.

Le brevet français n° 1.079.601 ne précise aucune caractéristique texturale: granulométrie, porosité, surface spécifique du graphite employé et outre une bonne capacité d'absorption la seule qualité requise se limite à un désbuilage parfait du solide. Les catalyseurs de ce brevet ont été testés sur des charges de 1,100 à 1,200 kg et dans ces conditions les productivités obtenues sont faibles, inférieures à 15 g d'oxyde d'éthylène par litre de catalyseur et par heure pour des essais à pression atmosphérique et de l'ordre de 115 g pour des essais sous 10 bars. Les sélectivités annoncées dans ce dernier cas sont médiocres: 60 à 68%.

Le brevet allemand n° 1.066.559 décrit très brièvement un seul essai dans un réacteur de 3 m de long et de 25 mm de diamètre. Le graphite artificiel employé n'est pas défini et les auteurs signalent

une faible productivité accompagnant une sélectivité mauvaise ne dépassant pas 55%.

Bien que testés uniquement sur des charges de 30 ml, donc dans des conditions plus défavorables que celles décrites ci-dessus, les nouveaux catalyseurs proposés se sont révélés nettement supérieurs. Avec des teneurs en argent beaucoup plus faibles que celles utilisées dans les précédents brevets: 100 à 250 g/litre au lieu de 75 à 500 g/l on obtient dans une série d'essais à pression atmosphérique des sélectivités de 70 à 76%, tandis que sous 20 bars les sélectivités atteignent 71% pour des productivités de 140 g d'oxyde d'éthylène par litre de catalyseur et par heure.

La préparation de ces nouveaux catalyseurs ne pose aucun problème et peut être réalisée par tout procédé classique. On peut en particulier opérer de façon connue en deux étapes par imprégnation ou enrobage d'un composé de l'argent en solution ou suspension dans un solvant volatil, suivi d'un traitement permettant le passage sur le support au métal.

Les composés d'argent utilisés peuvent être soit des sels: nitrate, formiate, lactate, citrate, carbonate, oxalate, silicylate, acétate, sulfate, propionate, maléate, malate, malonate, phtalate, tartrate, glycolate, succinate, oxyde, hydroxyde, acétylure ou céténure, soit des complexes de sels d'argent avec des molécules azotées, ammoniac, acrylonitrile, pyridine, éthanolamine, éthylène diamine, ou avec des $\beta$-dicétones. Les principaux solvants ou liquides de suspension employés sont l'eau, l'acétone, les alcools légers, l'éther, la pyridine, l'éthylène glycol, le diéthylène glycol ou les solvants chlorés.

Toutes les techniques qui permettent le passage de ces composés au métal ou à l'oxyde peuvent être appliquées en présence de graphite ou de matériaux graphités, par exemple la précipitation, la décomposition thermique en atmosphère inerte, oxydante ou réductrice et la réduction chimique. Un traitement particulièrement approprié est la décomposition thermique sur les supports de l'acétate d'argent dans les conditions suivantes: montée thermique de 20 à 280°C à raison de 20°C/heure suivie d'un palier de 10 à 30 heures à 280—300°C, la totalité de l'opération étant effectuée sous balayage d'azote avec addition possible d'oxygène ou d'hydrogène.

Il est possible d'ajouter à ces catalyseurs, dans les teneurs habituelles de 0 à 2% en poids, tous les promoteurs solides classiques de l'argent:

K, Ca, Cs, Ba, Pt, Ni, Sn, Cd, Sr, Li, Mg, Na, Rb,
Au, Cu, Zn, La, Ce, Th, Be, Sb, Bi, Ti, Pd, Ir,
Os, Ru, Fe, Al.

Ces éléments pouvant se trouver dans les catalyseurs finis, sous forme métallique ou sous forme d'oxyde ou de composé.

Il a été également observé que certains dérivés halogénés des hydrocarbures, ajoutés en faibles quantités aux réactifs, augmentent la sélectivité des catalyseurs mis au point par la demanderesse. L'emploi du dichloro-1,2 éthane, à une concentration maximale de 1 ppm par rapport au volume total gazeux se révélant particulièrement intéressant.

Les exemples 1 à 12 ci-dessous illustrent de façon non limitative la préparation et l'utilisation des catalyseurs selon l'invention. Les résultats obtenus dans ces exemples sont exprimés en taux de transformation globale de l'éthylène, en sélectivité et en productivité.

— taux de transformation globale de l'éthylène (T.T.G.):

$$\text{T.T.G.} = \frac{\text{Nombre de moles d'éthylène transformées}}{\text{Nombre de moles d'éthylène introduites}} \times 100$$

— sélectivité de la transformation en oxyde d'éthylène (SOE)

$$\text{S.O.E.} = \frac{\text{Nombre de moles d'oxyde d'éthylène formées}}{\text{Nombre de moles d'éthylène transformées}} \times 100$$

— la productivité (Pg)

$P_g$ = Nombre de grammes d'oxyde d'éthylène produit par litre de catalyseur et par heure.

Exemple 1

On place dans un ballon à solides, monté sur un évaporateur rotatif 42,5 g d'un graphite artificiel préparé par la Société Le Carbone Lorraine et dont les caractéristiques texturales sont rassemblées dans le tableau n° 1.

| | |
|---|---|
| Granulométrie | 3 mm (sphères) |
| Surface spécifique | 0,5 m2/g |
| Volume de porosité | 0,16 cm3/g |
| Répartition de la porosité en fonction du rayon des pores : | |
| 50 A° < R < 6,6 $\mu m$ | 32 % |
| 6,6 $\mu m$ < R < 57 $\mu m$ | 43 % |
| 57 $\mu m$ < R < 100 $\mu m$ | 25 % |
| Densité apparente | 1,47 |

TABLEAU N° 1

On porte la température du bain d'huile de l'évaporateur à 120°C et on laisse dégazer le support pendant une heure sous une pression partielle de 100 mm de mercure. Dans les mêmes conditions de température et de pression on introduit ensuite, goutte à goutte en 3 heures, 200 ml d'une solution pyridinique à 5% en poids d'acétate d'argent. Dans ces conditions l'évaporation du solvant est instantanés. Après introduction de la totalité de la solution, le support imprégné et séché, est transféré dans un réacteur tubulaire pour décomposer, dans un courant d'azote, l'acétate d'argent suivant la réaction connue:

$$4\ CH_3CO_2Ag \rightarrow 4\ Ag + 3\ CH_3CO_2H + CO_2 + C$$

Afin de contrôler thermiquement la réaction ce traitement est effectué avec une montée en température de 20°C/h jusqu'à un palier de 18 heures à 270°C. L'analyse indique que le catalyseur ainsi préparé contient 10% en poids d'argent.

Exemple 2

On place dans un ballon à solides monté sur un évaporateur rotatif 84 g d'un graphite artificiel préparé par la Société Le Carbone Lorraine et dont les caractéristiques sont rassemblées dans le tableau n° 2. On y ajoute 200 ml d'une solution à 10% en poids d'acétate d'argent dans la pyridine et on distille le solvant sous une pression partielle de 5 mm de mercure. Le support imprégné est séché, puis chargé dans un réacteur tubulaire pour décomposition de l'acétate d'argent dans les mêmes conditions que celles de l'exemple n° 1. On obtient ainsi produit contenant 10% d'argent en poids.

| | |
|---|---|
| Granulométrie | 500–700 $\mu m$ |
| Surface spécifique | 1,5 m2/g |
| Volume de porosité | 0,14 cm3/g |
| Répartition de la porosité en fonction du rayon des pores : | |
| 50 A° < R < 6,6 $\mu m$ | 85 % |
| R > 6,6 $\mu m$ | 15 % |
| Densité apparente | 1,60 |

TABLEAU N° 2

Exemple 3

On charge dans un réacteur de laboratoire travaillant à la pression atmosphérique 30 ml de

catalyseur préparé dans l'exemple n° 1. Le réacteur est constitué par un tube en acier inoxydable de 600 mm de long et de 16 mm de diamètre intérieur. Les réactifs sont admis par le bas et sont préchauffés sur un lit d'anneaux de porcelaine de 200 mm de haut, que supporte également la charge de catalyseur. Le chauffage de l'ensemble est assuré par une circulation d'huile dans une double enveloppe. Les gaz entrant et sortant du réacteur sont analysés en ligne à l'aide d'un chromatographe à double détection: un détecteur à ionisation de flamme pour l'oxyde d'éthylène, le méthane, le formol, le propylène, le propane, le méthanol et l'acétaldéhyde, et un détecteur à conductibilité thermique pour l'oxygène-azote, le dioxyde de carbone, l'éthylène et l'eau. Les deux colonnes de diamètre 1/8 de pouce et de longueur 2,5 mètres, montées en série sont remplies l'une de chromosorb 101, l'autre de porapak Q.

On fait passer sur le catalyseur un courant gazeux de 14 litres/heure constitué d'un mélange de 12% d'éthylène, 4,7% d'oxygène, 83,3% d'azote et 600 ppb de dichloro-1,2 éthane. Après 100 heures on obtient les résultats du tableau n° 3.

| T° C catalyseur | % T.T.G. | % S.O.E. | $P_g$ |
|---|---|---|---|
| 214 | 10 | 74 | 8 |
| 227 | 14 | 72 | 11 |

TABLEAU N° 3

Exemple 4.

A titre de comparaison avec les résultats de l'essai n° 3 précédent, on a testé sur le même appareillage et dans des conditions rigoureusement identiques un catalyseur industrialisé contenant 15% d'argent, sur un support silice-alumine. Les résultats obtenus figurent sur le tableau n° 4.

| T° C catalyseur | % T.T.G. | % S.O.E. | $P_g$ |
|---|---|---|---|
| 234 | 10 | 70 | 7,5 |
| 249 | 13 | 64 | 9 |

TABLEAU N° 4

Les sélectivités sont plus faibles et les activités inférieures sont mises en évidence par des températures de marche plus élevées pour des taux de transformation voisins.

Exemple 5

Dans le réacteur décrit dans l'exemple n° 3, on introduit une charge de 30 ml de catalyseur préparé dans l'exemple n° 2. Après un traitement d'activation de 30 heures consistant à faire passer sur le catalyseur un mélange air-éthylène 50%—50% à une température inférieure à 200°C, on introduit dans le réacteur un courant gazeux de 14 litres/heure constitué d'un mélange de 14% d'éthylène, 4,6% d'oxygène, 81,4 d'azote et 200 ppb de dichloro-1,2 éthane. Après 60 heures sous réactifs on obtient à 230°C un taux de transformation de l'éthylène de 7% avec une sélectivité en oxyde d'éthylène de 70%.

Exemple 6

On charge 30 ml du catalyseur préparé dans l'exemple n° 2 dans un réacteur de laboratoire fonctionnant sous pression et constitué essentiellement par un tube en acier inoxydable de 355 mm de long et 16 mm de diamètre intérieur, chauffé par un bain de nitrates fondus. Les réactifs admis par le bas du réacteur sont préchauffés sur un remplissage en porcelaine de 42 mm de hauteur. Le dispositif analytique est identique à celui décrit dans l'exemple n° 3.

On fait passer sur le catalyseur, à une pression de 20 bars, un courant gazeux contenant 13% d'éthylène, 5% d'oxygène et 82% d'azote, à un débit horaire spécifique de 9000 litres/heure normaux

par litre de catalyseur. Avec un taux de transformation de l'éthylène de 8,5% et une sélectivité en oxyde d'éthylène de 71%, on obtient à 221 °C une productivité an oxyde d'éthylène de 139 g par heure et par litre de catalyseur.

Exemple 7

A titre de comparaison avec les résultats de l'essai n° 6 précédent, on a testé sur le même appareillage et dans des conditions rigoureusement identiques le catalyseur commercialisé qui a déjà fait l'objet de l'essai n° 4. Il n'a pas été possible avec ce catalyseur de travailler dans les mêmes conditions de température. Au-dessus de 200°C on observe un emballement de la réaction se traduisant par une montés rapide de la température à 300°C et une consommation totale de l'oxygène entrant. A la température de 195°C, qui constitue la limite de contrôle thermique de la réaction, on obtient, pour un taux de transformation de l'éthylène de 3,5% et une sélectivité en oxyde d'éthylène de 69%, une productivité en oxyde d'éthylène de 59 g par heure et par litre de catalyseur.

Exemple 8

Suivant le mode opératoire décrit dans l'exemple n° 1 on prépare un catalyseur avec un graphite artificiel d'origine Le Carbone Lorraine, dont les caractéristiques sont rassemblées dans le tableau n° 5.

| | |
|---|---|
| Granulométrie | 4–5 mm |
| Surface spécifique | 0,10 m2/g |
| Volume de porosité | 0,18 cm3/g |
| Répartition de la porosité en fonction du rayon des pores : | |
| 50 A° < R < 6,6 μm | 6,5 % |
| 6,6 μm < R < 57 μm | 78 % |
| 57 μm < R < 100 μm | 15,5 % |
| Densité apparente | 1,73 |

TABLEAU N° 5

L'analyse indique que le catalyseur ainsi préparé contient 13% en poids d'argent.

Exemple 9

On charge 30 ml du catalyseur préparé dans l'exemple n° 8, dans le réacteur décrit dans l'exemple n° 3. Après un traitement d'activation identique à celui de l'exemple n° 5 on introduit à la pression atmosphérique un débit gazeux de 14 litres/heure formé d'un mélange de 14% d'éthylène, 4,6% d'oxygène, 81,4% d'azote et 120 ppb de dichloro-1,2 éthane. Après une vingtaine d'heures de marche, on obtient les résultats du tableau n° 6.

| T° C catalyseur | % T.T.G. | S.O.E. % |
|---|---|---|
| 212 | 5 | 74 |
| 231 | 10 | 70 |

TABLEAU N° 6

Exemple 10

Un exemple comparatif a été effectué sur un catalyseur préparé suivant le mode opératoire décrit dans l'exemple 1, avec un graphite artificiel de granulométrie de 4 à 5 mm et de volume total de

porosité faible formé uniquement de pores de rayons inférieurs à 6,6 microns. Les caractéristiques du graphite utilisé sont rassemblées dans le tableau n° 7.

| | |
|---|---|
| Granulométrie | 4–5 mm |
| Surface spécifique | 0,4 m2/g |
| Volume de porosité | 0,08 cm3/g |
| Répartition de la porosité en fonction du rayon des pores : | |
| 50 A° < R < 6,6 $\mu m$ | 100 % |
| R > 6,6 $\mu m$ | 0 % |
| Densité apparente | 1,76 % |

TABLEAU N° 7

L'analyse indique une teneur en argent du catalyseur de 11% en poids. On charge 30 ml de ce catalyseur dans le réacteur décrit dans l'exemple n° 3. Après traitement d'activation identique à celui de l'exemple n° 5, on introduit à la pression atmosphérique un débit gazeux de 14 litres/heure formé d'un mélange de 13% d'éthylène, 4,7% d'oxygène, 82,3% d'azote et 600 ppb de dichloro-1,2 éthane. Dans le domaine de température de 200 à 240°C l'activité de ce catalyseur exprimée par le taux de transformation de l'éthylène est faible. En poussant la température à 245°C on obtient un taux de transformation de l'éthylène de 2% avec une sélectivité en oxyde d'éthylène de 65%. Les performances de ce catalyseur sont nettement inférieures à celles obtenues dans les essais des exemples 3, 5, 6 et 9 avec les catalyseurs selon l'invention.

Exemple 11

Un exemple comparatif a été effectué sur un catalyseur préparé, suivant le mode opératoire décrit dans l'exemple 1, avec un graphite artificiel de granulométrie de 200 à 700 microns et de faible volume de porosité. Les caractéristiques du graphite utilisé sont rassemblées dans le tableau n° 8.

| | |
|---|---|
| Granulométrie | 200–700 $\mu m$ |
| Surface spécifique | 1,4 m2/g |
| Volume de porosité | 0,10 cm3/g |
| Répartition de la porosité en fonction du rayon des pores : | |
| 50 A° < R < 6,6 $\mu m$ | 100 % |
| R > 6,6 $\mu m$ | 0 % |
| Densité apparente | 1,73 |

TABLEAU N° 8

L'analyse indique une teneur en argent du catalyseur de 11% en poids. On charge 30 ml de ce catalyseur dans le réacteur décrit dans l'exemple n° 3. Après un traitement d'activation de 50 heures par un mélange air-éthylène 50%—50% à une température de 175 à 215°C, on introduit dans le réacteur un courant gazeux de 14 litres/heure formé d'un mélange de 14% d'éthylène, 4,8% d'oxygène, 81,2% d'azote et 200 ppb de dichloro-1,2 éthane. Les meilleurs résultats obtenus figurent sur le tableau n° 9.

| T° C catalyseur | % T.T.G. | % S.O.E. |
|---|---|---|
| 210 | 4 | 59 |
| 217 | 5 | 52 |

TABLEAU N° 9

Les performances de ce catalyseur sont nettement inférieures à celles obtenues dans les exemples 3, 5, 6 et 9 avec les catalyseurs selon l'invention.

Exemple 12

On charge 30 ml du catalyseur préparé dans l'exemple 8, dans le réacteur décrit dans l'exemple 3. On fait passer sur le catalyseur, à la pression atmosphérique, un débit gazeux de 13,5 l/heure, constitué d'un mélange de 50% de propylène, 10% d'oxygène et 40% d'azote. A 260°C on obtient une sélectivité en oxyde de propylène de 25,7% pour une conversion globale du propylène de 1,1%.

Exemple 13

Un exemple comparatif a été effectué sur un catalyseur de teneur en argent 12,4%, préparé suivant le mode opératoire décrit dans l'exemple 1, avec un graphite artificiel en grains de diamètre 400 à 500 millimicrons, de surface spécifique 10 m2/g et de volume total de porosité 0,085 cm3/g. La surface spécifique de ce catalyseur est de 7 m2/g.

On fait passer sur le catalyseur à une pression de 20 bars et à une température de 276°C un courant gazeux contenant 5% d'éthylène, 5% d'oxygène et 90% d'azote à un débit horaire spécifique de 9.000 l/h/normaux par litre de catalyseur. Le taux de transformation de l'éthylène est de 4,7% et la sélectivité en oxyde d'éthylène de 36%, ce qui montre qu'une surface spécifique du catalyseur inférieure à 10 m2/g n'est pas une caractéristique suffisante pour qu'un graphite constitue un bon support de l'argent.

## Revendications

1. Catalyseurs à base d'argent sur supports de graphite artificiel poreux ou de matériaux graphités poreux, destinés à la synthèse en phase vapeur des époxydes par réaction d'un hydrocarbure éthylénique avec de l'oxygène ou des mélanges gazeux en contenant, caractérisés en ce que les supports graphitiques présentent:
   - —une surface spécifique inférieure à 10 $m^2/g$,
   - —une granulométrie de 50 $\mu m$ à 10 mm,
   - —un volume total de porosité compris entre 0,1 et 0,4 $cm^3/g$,
   - —une répartition de la porosité telle que les pores de diamètre inférieur à 6,6 $\mu m$ représentent au plus 60% du volume total de la porosité lorsque la granulométrie du support est supérieure â 0,7 mm, et au plus 90% du volume total de la porosité lorsque la granulométrie du support est inférieure ou égale à 0,7 mm.
2. Catalyseurs selon la revendication 1, caractérisés en ce qu'ils contiennent de 5 à 20% en poids d'argent et qu'ils sont préparés par une imprégnation du support graphitique au moyen d'une solution d'un sel d'argent, cette imprégnation étant suivie d'une décomposition du sel libérant le métal.
3. Procédé de préparation d'epoxyde par réaction d'oxygène ou de mélange gazeux contenant de l'oxygène sur un hydrocarbure éthylénique, caractérisé par l'emploi d'un catalyseur selon l'une des revendications 1 ou 2.
4. Procédé de préparation de l'oxyde d'éthylène à partir de l'éthylène selon la revendication 3.
5. Procédé selon l'une des revendications 3 et 4, caractérisé en ce que l'on opère en présence d'un composé organique halogéné.
6. Procédé selon la revendication 5, caractérisé en ce que le composé organique halogéné utilisé est le dichloro-1,2-éthane.

## Claims

1. Catalysts based on silver supported on porous artificial graphite or on porous graphite-containing materials, the catalysts being intended for the vapour phase synthesis of epoxides by reaction of an ethylenic hydrocarbon with oxygen or with gaseous mixtures containing oxygen, characterised in that the graphite-type supports have:

a specific surface area of less than 10 $m^2/g$,

a particle size of 50 μm to 10 mm,

a total porosity volume of between 0.1 and 0.4 $cm^3/g$, and

a porosity distribution which is such that the pores of diameter less than 6.6 μm represent at most 60% of the total porosity volume if the particle size of the support is greater than 0.7 mm, and at most 90% of the total porosity volume if the particle size of the support is less than or equal to 0.7 mm.

2. Catalysts according to Claim 1, characterised in that they contain from 5 to 20% by weight of silver and that they are prepared by impregnating the graphite-type support with a solution of a silver salt, this impregnation being followed by decomposition of the salt, liberating the metal.

3. Process for the preparation of an epoxide by reaction of oxygen or of a gaseous mixture containing oxygen with an ethylenic hydrocarbon, characterised by the use of a catalyst according to one of Claims 1 or 2.

4. Process for the preparation of ethylene oxide from ethylene in accordance with Claim 3.

5. Process according to one of Claims 3 and 4, characterised in that it is carried out in the presence of a halogen-containing organic compound.

6. Process according to Claim 5, characterised in that the halogen-containing organic compound used is 1,2-dichloroethane.

**Patentansprüche**

1. Für die Synthese von Epoxiden durch Umsetzung eines äthylenischen Kohlenwasserstoffs mit Sauerstoff oder sauerstoffhaltigen Gasgemischen in der Dampfphase bestimmte Katalysatoren auf Basis von Silber auf Trägern aus porösem künstlichem Graphit oder porösen graphitisierten Materialien, dadurch gekennzeichnet, daß die graphitischen Träger

eine spezifische Oberfläche von weniger als 10 $m^2/g$,

eine Korngröße von 50 μm bis 10 mm,

ein Gesamtporenvolumen zwischen 0,1 und 0,4 $cm^3/g$ und

eine solche Verteilung der Porosität aufweisen, daß die Poren mit einem Durchmesser von weniger als 6,6 μm höchstens 60% des gesamten Porenvolumens ausmachen, wenn die Korngröße des Trägers über 0,7 mm liegt, und höchstens 90% des gesamten Porenvolumens ausmachen, wenn die Korngröße des Trägers unter oder bei 0,7 mm liegt.

2. Katalysatoren nach Anspruch 1, dadurch gekennzeichnet, daß sie 5 bis 20 Gew.-% Silber enthalten und durch Imprägnierung des graphitischen Trägers mit einer Lösung eines Silbersalzes und anschließende Zersetzung des Salzes unter Freisetzung des Metalls hergestellt worden sind.

3. Verfahren zur Herstellung von Epoxid durch Umsetzung von Sauerstoff oder eines Sauerstoff enthaltenden Gasgemisches mit einem äthylenischen Kohlenwasserstoff, dadurch gekennzeichnet, daß man einen Katalysator gemäß Anspruch 1 oder 2 verwendet.

4. Verfahren zur Herstellung von Äthylenoxid aus Äthylen gemäß Anspruch 3.

5. Verfahren nach einem der Ansprüche 3 und 4, dadurch gekennzeichnet, daß man in Gegenwart einer halogenierten organischen Verbindung arbeitet.

6. Verfahren nach Anspruch 5, dadurch gekennzeichnet, daß die verwendete halogenierte organische Verbindung Dichlor-1,2-äthan ist.